# EUROPÄISCHE PATENTSCHRIFT

(11) **EP 2 722 657 B1**
(45) Veröffentlichungstag und Bekanntmachung des Hinweises auf die Patenterteilung: **21.12.2016**
(21) Anmeldenummer: 13188095.7
(22) Anmeldetag: 10.10.2013
(51) Int. Cl.: G01G 19/44

(54) **Patientenwaage mit Kameraüberwachung und Dialysetherapiesystem mit kameraüberwachtem Wiegeablauf**
Patient weighing scales with camera monitoring and dialysis therapy system with camera monitored weighing process
Pèse-personne avec surveillance par caméra et système de thérapie par dialyse comprenant un processus de pesée surveillé par caméra

(30) Priorität: 16.10.2012 DE 102012109861
(43) Veröffentlichungstag der Anmeldung: 23.04.2014
(73) Patentinhaber: B. Braun Avitum AG, 34212 Melsungen (DE)
(72) Erfinder: Bröker, Björn, 34355 Staufenberg (DE); Ritter, Kai-Uwe, 91126 Rednitzhembach (DE)
(74) Vertreter: Winter, Brandl, Fürniss, Hübner, Röss, Kaiser, Polte - Partnerschaft mbB

(56) Entgegenhaltungen:
- WO-A1-2006/104903
- WO-A2-2007/146220
- FR-A1- 2 256 399
- US-A1- 2011 066 006
- Tietz: "Convenient solutions for precise results", Internet , 18. Januar 2006 (2006-01-18), XP002717215, Gefunden im Internet: URL:http://elhospital.com/eh/formas/54045/ MedicalLine_Int_Engl_low.pdf [gefunden am 2013-11-29]
- Anonymous: "Bed Scale 7711 Operating Manual", www.soehnle-professional.com , 6. Juli 2010 (2010-07-06), XP002717216, Gefunden im Internet: URL:http://www.soehnle-professional.com/up loads/tx_soehnleproductsmanuals/BAL_7711_G B_01.pdf [gefunden am 2013-11-29]

## Beschreibung

Die Erfindung betrifft eine Gewichtserfassungseinrichtung mit einer Patientenwaage und Videoüberwachung eines Wiegeablaufs mittels einer Kamera im Rahmen einer Dialysetherapie und ein Dialsysetherapiesystem mit kameraüberwachtem Wiegeablauf, und bezieht sich insbesondere auf eine Patientenwaage als eine Komponente der Gewichtserfassungseinrichtung, die dazu ausgelegt ist, in Verbindung mit einer Kamera einen Wiegevorgang eines Patienten im Rahmen einer Dialysebehandlung aufzuzeichnen, sowie auf ein zur Dialysetherapie bei Niereninsuffizienz verwendetes System mit einer solchen Waage.

Für Dialysetherapien oder Dialysebehandlungen sind häufige Wiegevorgänge vor, während und nach der eigentlichen Dialyse durchzuführen. Bei Dialysepatienten liegt eine Unterfunktion der natürlichen Niere vor. Deren Aufgaben, etwa die Entfernung urämischer Toxine und die Ausscheidung von überschüssigem Körperwasser, muss sodann eine künstliche Niere (ein Dialysator) übernehmen. In einer Ausführungsform der künstlichen Niere presst diese während ihres Blutreinigungsvorgangs Plasmawasser des Patienten über eine Dialysatormembran ab. Dieses Abpressen von Plasmawasser, oder diese Wasserentfernung durch Filtration des Bluts über eine Siebmembran mit einem Druck von 0 bis 1 bar und mit dabei auftretenden Mitnahmeeffekten (Konvektion) zur selektiven Entgiftung, wird als Ultrafiltration bezeichnet. Mittels der Ultrafiltration ist es möglich, Teilchen im Bereich der Größe mittelmolekularer Substanzen (etwa 0,1 bis 0,05 µm), wie beispielsweise Endotoxine, Viren, Kolloide, Proteine und Enzyme aus dem Blut des Patienten zu entfernen bzw. zu waschen und es dadurch zu reinigen. Da diese Reinigung außerhalb des Körpers des Patienten erfolgt, verliert der Körper dabei zugleich Wasser und beispielsweise Elektrolyte, die ersetzt werden müssen. Ferner können je nach Durchlässigkeit der Filter große Filtratmengen in kurzer Zeit entstehen, die den notwendigen Volumenentzug des Patienten übersteigen. Eine der Filtratmenge entsprechende Substitution von Flüssigkeit physiologischer Zusammensetzung ist daher erforderlich, um den Flüssigkeitshaushalt des Patienten zu stabilisieren.

Im Gegensatz zu Menschen mit gesunden Nieren ist bei Nierenkranken die Fähigkeit zur Regulierung des Wasserhaushalts im Körper mehr oder weniger stark gestört, so dass überschüssiges Wasser im Körper zurückgehalten wird. Dadurch kommt es zu einer Mehrbelastung des Organismus als einer der Hauptfaktoren, die für Dialysepatienten eine Zunahme an Komplikationen und eine Abnahme an Lebenserwartung bedeuten. Eine der wichtigsten Aufgaben der Dialysebehandlung besteht daher darin, die fehlende Regulierung des Wasserhaushalts so gut wie möglich zu ersetzen. Üblicherweise wird dazu bei Dialysebehandlungen ein sogenanntes Trockengewicht als Behandlungsziel festgelegt. Ein Definitionsvorschlag für das Trockengewicht zieht einen Schätzwert für das Gewicht heran, das ein Dialysepatient hätte, wenn er/sie keine Nierenerkrankung hätte. Medizinisch gesehen wird als Trockengewicht das Gewicht zum Ende der Dialyse bezeichnet, bei dem der Patient keine Überwässerungsanzeichen aufweist, frei von Symptomen ist, sich wohlfühlt und bei dem bis zur nächsten Dialyse ein normaler Blutdruck erhalten bleibt.

Zur Bestimmung des Trockengewichts, das als solches nicht durch das Ergebnis eines objektiven Vorgangs einfach festgelegt werden kann, sind möglichst exakte Wiegevorgänge des Patienten vor und nach der Dialysebehandlung unerlässlich, um die Ultrafiltrationsmenge so bemessen zu können, dass als Sollgewicht das Trockengewicht möglichst genau erreicht wird. Aus Beobachtungen ist aber bekannt, dass immer wieder unerklärliche und unerwünschte Abweichungen der Ultrafiltrationsmenge beim Wiegen oder zwischen einzelnen Wiegevorgängen festgestellt werden. Um in solchen Fällen ausschließen zu können, dass die Dialysemaschinen die Ursache solcher Abweichungen sind, müssen diese zeitaufwändig und kostenträchtig außer Betrieb genommen und in einer simulierten Behandlung auf ihre Genauigkeit geprüft werden. Häufig stellt sich dann heraus, dass die Funktion der Maschine nicht zu beanstanden ist.

Die Ursache für eine Abweichung kann dann beispielsweise auf derzeit noch nicht nachvollziehbare Ursachen zurückzuführen sein. Beispiele für eine Vielzahl derartiger Ursachen sind, dass sich der Patient zwischen Wiegevorgängen erleichtert hat, dies aber nicht ordnungsgemäß vermerkt wurde; der Patient absichtlich ein falsches Gewicht meldet, mit dem Ziel, sich mehr Flüssigkeit entziehen zu lassen, um danach mehr als zugestanden trinken zu können; eine Nahrungs- und/oder Getränkeaufnahme nicht protokolliert wurde; eine während der Behandlung verabreichte Infusion nicht genau gemessen und/oder nicht protokolliert wurde; der Patient bei zwei einander zuzuordnenden Wiegevorgängen nicht dieselbe Kleidung trägt; der Patient bei zwei einander zuzuordnenden Wiegevorgängen unterschiedliche Rollstühle mit jeweils anderem Gewicht benutzt; beim Wiegen kurzzeitig Gegenstände (eine Tasche, ein Mobiltelefon oder dergleichen) abgelegt oder nicht abgelegt wurden; ein benutzter Rollstuhl einen nicht optimalen Stand hatte; oder sich der Patient während des Wiegevorgangs abstützt.

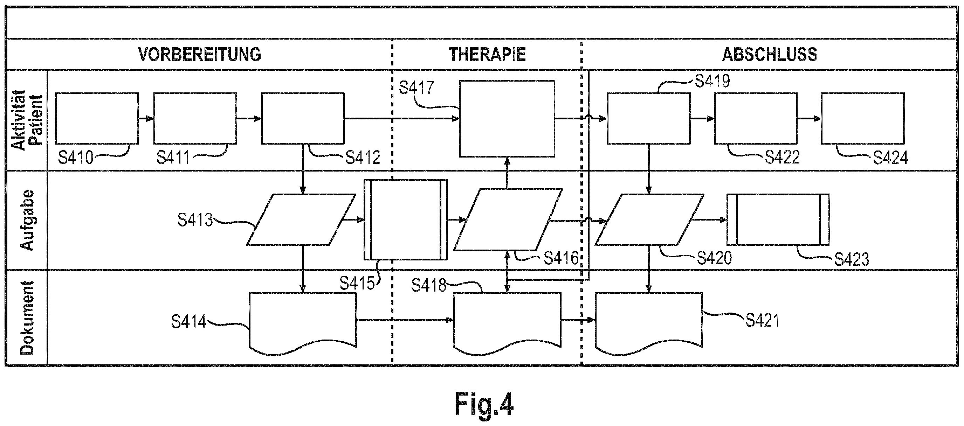

Fig. 4 zeigt beispielsweise eine vereinfachte Darstellung eines bekannten Ablaufs eines Wiegevorgangs unter Verwendung einer Protokollierung erfasster Daten auf Papier. Im Einzelnen sind in dem gezeigten zeitlichen Ablauf in chronologischer Abfolge Schritte oder Aktivitäten angegeben, die während des Wiegevorgangs eines Dialysepatienten im Rahmen eines Therapieablaufs mit vorbereitendem Wiegen vor der Therapie, der Therapie basierend auf den während des Wiegens ermittelten Daten, und dem Abschluss der Therapie mit erneutem kontrollierendem Wiegen von dem Patienten und Personal einer Dialysestation durchzuführen sind. Insoweit Aktivitäten und/oder Schritte parallel und/oder gleichzeitig durchzuführen sind, ist die Nummerierung der einzelnen Schritte nicht zwingend als Abfolge oder Aufeinanderfolge zu verstehen, sondern kann aus Zweckmäßigkeitsgründen auch nur eine Angabe von Bezugszeichen zur eindeutigen Zuordnung darstellen.

Die in Fig. 4 während des zeitlichen Ablaufs des Wiegevorgangs von links nach rechts untereinander in den einzelnen Zeilen angegebenen Bezeichnungen, d. h. "Aktivität Patient", "Aufgabe" und "Dokument", können eine variable Zuordnung von Aktivitäten zu Patient und/oder Personal beinhalten. Beispielsweise kann ein Patient eine Protokollierungsaktivität durchführen, oder kann Personal diese Protokoll ierungsaktivität durchführen.

Gemäß Fig. 4 beginnt ein Dialysetherapieablauf damit, dass ein Patient in einem Schritt S410 im Dialysezentrum eintrifft. In einem Schritt S411 legt er zumindest teilweise seine Kleidung, beispielsweise eine Jacke, und gegebenenfalls mitgeführtes Gepäck in einer zum Erhalt einer definierten Ausgangssituation üblicherweise vorbestimmten Weise ab. In einem Schritt S412 erfolgt der Wiegevorgang, während dem in einem Schritt S413 ein derzeitiges Ist-Gewicht des Patienten ermittelt wird. Hierbei kann sich der Patient selbst wiegen oder sich von Personal wiegen lassen. In einem Schritt S414 wird dieses Ist-Gewicht durch beispielsweise Personal der Dialysestation in ein Wiegeprotokoll eingetragen. Das Wiegeprotokoll wird während einer Dialysetherapie fortgeführt und kann in deren Rahmen durchgeführte mehrere Wiegevorgänge beinhalten. Darüber hinaus erfolgt die Protokollierung von Therapiedaten und somit auch Wiegevorgängen über mehrere zeitlich getrennte Dialysebehandlungen hinweg. Nach der Ermittlung des Ist-Gewichts des Patienten in Schritt S413 wird dieses die Dialysebehandlung vorbereitend dazu herangezogen, in einem Schritt S415 eine Soll-Ultrafiltrationsmenge bzw. ein aktuelles Ist-Trockengewicht zu bestimmen. Darauf basierende Therapiedaten bzw. Behandlungsparameter werden sodann in einem Schritt S416 von Personal in eine Dialysemaschine eingegeben bzw. in diese übertragen, und in einem Schritt S417 wird mit diesen Therapiedaten nach dem Anschluss des Patienten an die Dialysemaschine die Dialysebehandlung mit den festgelegten bzw. verschriebenen Therapiedaten durchgeführt. Möglichst gleichzeitig bzw. parallel dazu wird der Therapieablauf oder Therapieverlauf von Personal in einem Schritt S418 mitprotokolliert. Nach dem Ende der Dialysetherapie wird der Patient von der Dialysemaschine abgeschlossen und sodann in einem Schritt S419 erneut gewogen. Erneut werden während des Wiegevorgangs in einem Schritt S420 möglichst zeitgleich oder parallel dazu das Ist-Gewicht bzw. das Ist-Trockengewicht des Patienten ermittelt und in einem Schritt S421, im Rahmen einer Dokumentation der Behandlung, wieder in das mitgeführte Protokoll eingetragen und/oder in einem Schritt S423 in eine Datenbank, beispielsweise eine elektronische Patientendatenbank oder Patientenakte, eingetragen. Nach dem Wiegevorgang kann sich der Patient in einem Schritt S422 wieder ankleiden und sodann in einem Schritt S424 das Dialysezentrum verlassen.

Da bei der während des Therapieablaufs gemäß Fig. 4 durchgeführten rein manuellen Dokumentation das Personal neben der Betreuung der Patienten noch die Aufgabe hat, die Dokumentation durchzuführen, d. h. auf Papier auszufüllen und/oder in andere elektronische Geräte zu übertragen, ist es aufgrund der vielen zeitlich konzentrierten Abläufe für das Personal schwierig, sich an Einzelheiten zu erinnern. Die manuelle Dokumentation der Therapie durch Personal und/oder den Patienten selbst unterliegt daher unter anderem den vorstehend beispielhaft genannten Fehlermöglichkeiten und dadurch bedingten Abweichungen.

Von der Anmelderin selbst existiert ein aus dem Stand der Technik bekanntes System zur Überwachung und mit einer Datenbank. Dieses Überwachungssystem ist eine Software zur transparenten Abbildung und Steuerung zahlreicher Vorgänge einer Dialysebehandlung. Die generierten Daten angeschlossener Dialysegeräte, Analysegeräte (z. B. Blutgasanalyse) und Patientenwaagen werden automatisch in das Überwachungssystem übertragen und gespeichert. Dort werden sie visualisiert und stehen zur Bearbeitung zur Verfügung. Durch den bidirektionalen Datentransfer zwischen dem bekannten Überwachungssystem und den angeschlossenen Dialysegeräten sind die Datensätze auch während der Behandlung konsistent und abrufbar.

Ein Dokument, welches im Internet unter der URL http://www.elhospital.com/eh/formas/54045/MedicalLine Int Engl low.pdf gefunden werden kann, offenbart Gewichtsbestimmungseinrichtungen (Waagen), die auch für Dialysebehandlungen eingesetzt werden können. Diese Waagen bieten allerdings keinen Schutz oder Nachweis hinsichtlich systematischer, zufälliger oder absichtlicher Fehlwägung.

Die WO 2007/146220 A2 offenbart ein Videoüberwachungssystem mit mehreren Kameras, welches dazu ausgelegt ist, den Verlauf der Pflege eines bettlägerigen Patienten optisch aufzuzeichnen, abzuspeichern und in einem Datennetz abrufbar zu machen. Ziel ist die Reduzierung von Papierarbeit bei der Dokumentation des Pflegevorgangs.

Die US 2011/0066006 A1 offenbart ein System zur kontinuierlichen Überwachung eines Patienten bei der Hämodialyse. Insbesondere ist die kontinuierliche Blutdruckmessung während der Dialyse offenbart, wobei der Patient einen Sensor mit sich trägt, der die entsprechenden Daten drahtlos auf einen Controller überträgt. Zusätzlich wird die Messung des Körpergewichtes vor und nach der Dialyse über eine drahtlos an das System angebundene Waage offenbart, da die Gewichtsbestimmung hinsichtlich der abgezogenen Flüssigkeitsmenge von Wichtigkeit für die Behandlung ist.

Solche bekannten Anordnungen auf diesem Gebiet können durch eine Vernetzung von Einzelsystemen wie beispielsweise Maschinen, Waagen und Medikation zwar eine gewisse Unterstützung des Ablaufs auf einer Dialysestation bereitstellen. Sie sind nachteilig jedoch nicht in der Lage, nachvollziehbaren Aufschluss über während des Wiegens von Patienten auftretende Abweichungen ermittelter Patientengewichte und/oder Ultrafiltrationsmengen zu geben.

### Kurze Beschreibung der Erfindung

Der Erfindung liegt daher als eine Aufgabe zugrunde, eine Patientenwaage und ein Dialysetherapiesystem mit einer Patientenwaage zu schaffen, mit welchen ein zur Dialysebehandlung eines Patienten erforderlicher Wiegevorgang zur Ermittlung des Patientengewichts im Hinblick auf Ursachen auftretender Abweichungen nachvollziehbar durchgeführt werden kann, und welche es erlauben, auftretende Abweichungen zu verringern und während der Dialysebehandlung zu berücksichtigende Ultrafiltrationsmengen genauer zu bestimmen.

Diese Aufgabe wird erfindungsgemäß durch die Merkmalskombination des Anspruchs 1, alternativ durch die Merkmalskombination des Anspruchs 10 gelöst.

Solche bekannten Anordnungen auf diesem Gebiet können durch eine Vernetzung von Einzelsystemen wie beispielsweise Maschinen, Waagen und Medikation zwar eine gewisse Unterstützung des Ablaufs auf einer Dialysestation bereitstellen. Sie sind nachteilig jedoch nicht in der Lage, nachvollziehbaren Aufschluss über während des Wiegens von Patienten auftretende Abweichungen ermittelter Patientengewichte und/oder Ultrafiltrationsmengen zu geben.

### Kurze Beschreibung der Erfindung

Der Erfindung liegt daher als eine Aufgabe zugrunde, eine Patientenwaage und ein Dialysetherapiesystem mit einer Patientenwaage zu schaffen, mit welchen ein zur Dialysebehandlung eines Patienten erforderlicher Wiegevorgang zur Ermittlung des Patientengewichts im Hinblick auf Ursachen auftretender Abweichungen nachvollziehbar durchgeführt werden kann, und welche es erlauben, auftretende Abweichungen zu verringern und während der Dialysebehandlung zu berücksichtigende Ultrafiltrationsmengen genauer zu bestimmen.

Diese Aufgabe wird erfindungsgemäß durch die Merkmalskombination des Anspruchs 1, alternativ durch die Merkmalskombination des Anspruchs 10 gelöst. Vorteilhafte Weiterbildungen der Erfindung sind Gegenstand der beigefügten Unteransprüche.

Ein Grundgedanke der Erfindung besteht somit darin, den Vorgang "Wiegen vor und nach der Dialyse, sowie während der eigentlichen Dialysebehandlung", lückenlos per Video zu erfassen und auszuwerten. Es wird dazu eine Anordnung zur verbesserten Überwachung des Patientengewichts vor, während und nach dem Dialysevorgang vorgeschlagen, wozu ggf. vernetzte, an der Waage und/oder der Dialysemaschine und/oder in deren Umgebung angeordnete Videokameras bereitgestellt werden.

Insbesondere kann eine Patientenwaage, die Teil einer Gewichtsermittlungsvorrichtung und/oder Gewichtserfassungseinrichtung bildet, mit initiiert werden. Die Gestensteuerung kann sich auf den gesamten Ablauf des kameragestützt überwachten Wiegevorgangs erstrecken

Ferner kann eine Dialysemaschine, welche durch Zusammenwirken mit der Patientenwaage zumindest Teil eines Gewichtsüberwachungssystems bilden kann, mit wenigstens einer daran oder an einem anderen geeigneten Ort angebrachten Kamera vorgesehen sein. Der Patient wird während des Dialysevorgangs mittels der Kamera beobachtet. Vorzugsweise ist die Kamera über ein Netzwerk mit einem Überwachungssystem verbunden. Der gesamte Vorgang kann dann im Überwachungssystem gespeichert werden. Programmroutinen analysieren aus gespeicherten Daten Bewegungen des Patienten und erkennen Nahrungsaufnahmen und/oder Flüssigkeitsaufnahmen. Eine Qualitätssicherung von Maßnahmen während der Dialyse kann durchgeführt werden, etwa in Bezug darauf, ob Medikamente verabreicht und auch eingenommen wurden usw. Das Bild der Kamera kann auch als eine Direktübertragung ("Online"- oder Live-Übertragung) genutzt werden, um nach dem Patienten zu sehen. Das Bild der Kamera kann darüber hinaus auch für eine Videotelefonie-Verbindung mit dem Patienten genutzt werden. Ferner kann das System etwa einen Gang des Patienten zu Waschräumen, Toiletten und dergleichen erkennen und ermitteln, ob ein entsprechender Wiegevorgang dazu durchgeführt wird oder wurde.

Insgesamt werden erfindungsgemäß Vorteile dahingehend erzielt, dass (nur) bei unerklärlichen Abweichungen des Gewichts vor und nach der Dialyse die Video-Aufzeichnungen ausgewertet werden (können), und dass mittels einer automatischen Auswertung der Videosequenzen relevante Szenen herausgefiltert werden (können) und es dem Personal ermöglicht wird, diese zu bewerten und ein gegebenenfalls vorliegendes Fehlverhalten des Patienten auszuwerten und zu korrigieren. Patienten mit regelmäßigen Abweichungen können im System gespeichert werden, so dass das System darauf basierend einen Hinweis geben kann, dass der Wiegevorgang nicht selbstständig vom Patienten bzw. nur unterstützt durch oder im Beisein von Personal durchgeführt werden darf.

Dazu wird insgesamt eine Anordnung zur verbesserten Überwachung des Patientengewichts vor, während und nach dem Dialysevorgang unter Verwendung von vernetzten, an einer Waage und/oder einer Dialysemaschine oder in einem Raumbereich mit Blick auf zu erfassende Information liefernde Abschnitte derselben angebrachten Videokameras vorgeschlagen.

Die Aufgabe wird somit gelöst durch eine Gewichtserfassungseinrichtung zur Ermittlung eines Patientengewichts für eine Dialysebehandlung, gekennzeichnet durch eine Patientenwaage, die dazu angeordnet ist, einen Wiegevorgang durchzuführen und dadurch ein Ist-Gewicht eines sich auf der Waage befindenden Patienten zu erfassen; optional einen Netzwerkanschluss, der dazu angeordnet ist, zumindest eine Information und/oder Daten betreffend die Ermittlung des Patientengewichts und/oder den Wiegevorgang erzeugende Komponente der Gewichtserfassungseinrichtung mit einem Datennetzwerk zu verbinden; eine Ausgabeeinrichtung, die dazu angeordnet ist, das erfasste Ist-Gewicht des Patienten an der Patientenwaage ablesbar und/oder in einem vorbestimmten Datenformat in das Datennetzwerk auszugeben oder alternativ/zusätzlich hierzu einen ggf. lösbaren Datenspeicher; und zumindest eine Bildinformationsaufnahmeeinrichtung, die ein Sichtfeld auf die Patientenwaage zur visuellen Erfassung von Wiegevorgängen aufweist und dazu angeordnet ist, innerhalb des Sichtfelds einen Ablauf des Wiegevorgangs derart aufzuzeichnen, dass eine Ursache einer im Verlauf der Dialysetherapie auftretenden Änderung des Ist-Gewichts aus zumindest einer entsprechenden Aufzeichnung zumindest eines Wiegevorgangs ermittelbar ist.

Vorteilhaft ist dabei die Bildinformationsaufnahmeeinrichtung eine Kamera vorzugsweise mit Anschluss an das Datennetzwerk, die in die Patientenwaage eingebaut ist.

Vorteile einer solchen Kamera, die allgemein analog und/oder digital, bevorzugt aber digital aufzeichnen kann, bestehen unter anderem darin, dass die Kamera in ihren Eigenschaften, beispielsweise im Hinblick auf eine erforderliche Auflösung, einen zweckmäßigen Teleobjektivbereich und eine Vergrösserbarkeit/ Verkleinerbarkeit von Teilen einer Aufzeichnung, ein erzeugtes Video- und/oder Bildformat und dergleichen, gut auf die Gegebenheiten und Anforderungen der Patientenwaage abstimmbar und in diese einbindbar ist, und dass bei einer Verlagerung der Patientenwaage die Kamera ortsfest mitbewegt wird und Einstellvorgänge nach der Verlagerung auf ein Minimum reduziert werden können.

Alternativ kann die Bildinformationsaufnahmeeinrichtung eine Kamera vorzugsweise mit Anschluss an das Datennetzwerk und außerhalb der Patientenwaage an einer Dialysemaschine angeordnet sein.

Vorteile bestehen hierbei unter anderem darin, dass die Patientenwaage innerhalb eines voreingestellten Sichtbereichs der Kamera verlagert werden kann, beispielsweise wenn Patienten von Personal gestützt werden müssen und dazu die Waage verschoben werden soll, oder ein bisheriger Standort der Personenwaage korrigiert werden muss oder durch einen Stoß gegen dieselbe unabsichtlich bewegt wird, ohne dass dann Änderungen an der Kamera vorgenommen werden müssen. Außerdem muss bei einem Ausfall einer Kamera die Dialysebehandlung nicht unterbrochen werden, wenn auf der Dialysestation nur eine Patientenwaage, aber mehrere mit Kameras versehene Dialysegeräte zur Verfügung stehen. Die Patientenwaage muss dann nur in den Sichtbereich einer anderen Kamera gebracht werden.

Vorteilhaft ist auch, wenn die Bildinformationsaufnahmeeinrichtung in Bezug auf ihr aufzeichenbares Sichtfeld verschwenkbar ist.

Dadurch wird es möglich, etwa einen Gesamtsichtbereich einer Kamera in mehrere Bereiche zu unterteilen und die Kamera ablaufgesteuert auf einen momentan jeweils benötigten Teilbereich zu richten bzw. scharfzustellen, oder einen solchen Bereich für eine Nahaufnahme heranzuholen oder für eine Übersichtsaufnahme zu erweitern. Denkbar ist auch, dass dadurch der Einsatz nur einer Kamera für mehrere Patientenwaagen erfolgt, wobei die Kamera für Wiegevorgänge jeweils zwischen zugeordneten Waagen hin und her verschwenkt wird.

Bevorzugt ist die Bildinformationsaufnahmeeinrichtung durch Gesten einer sich im Sichtfeld befindenden Person aktivierbar, verschwenkbar und/oder deaktivierbar, und/oder sind ein Beginn, eine Unterbrechung und/oder ein Ende einer Aufzeichnung durch Gesten einer sich im Sichtfeld befindenden Person steuerbar.

Hierdurch kann entweder die zu wiegende Person selbst eine Aufnahme steuern, beispielsweise für einen längeren Gang zu Waschräumen unterbrechen, oder beenden, falls für eine längere Zeit kein entsprechend ausführendes Personal zur Verfügung steht. Oder kann eine beispielsweise die zu wiegende Person stützende Pflegekraft die Aufnahme bequem steuern, ohne die Unterstützung der Person wesentlich ändern zu müssen. Insgesamt ist die Gestensteuerung auch eine effiziente Art der Steuerung, die gegenüber einer langsameren Steuerung über Bedientasten Speicherplatz einspart und/oder bei einer Speicherung der Aufnahme im Datennetzwerk den Datenverkehr auf dem Datennetzwerk reduziert.

Vorteilhaft ist die Bildinformationsaufnahmeeinrichtung dazu angeordnet, zumindest außerhalb eines aufzuzeichnenden Wiegevorgangs ein Direktbild ihres Sichtbereichs zu erfassen und bereitzustellen.

Durch ein solches auch als Live-Bild bezeichnetes Direktbild, bei dem noch keine speichernde Aufnahme erfolgt, ist der Sichtbereich der Kamera permanent auf beispielsweise einem Bildschirm überwachbar. Diese Art der Überwachung erfolgt bevorzugt in Echtzeit. Es kann dadurch zum Beispiel aus einer gewissen Entfernung zur Waage, etwa wenn wenig Personal eine größere Anzahl von Patienten zu betreuen hat und sich auf der Dialysestation bewegen muss, beobachtet werden, ob ein Patient bereits für einen Beginn eines Wiegevorgangs bereit ist, oder ob versucht wird, die Waage und/oder den Wiegevorgang in einer unerwünschten und Messergebnisse möglicherweise verfälschenden Weise zu manipulieren.

Bevorzugt beinhaltet die Gewichtserfassungsvorrichtung eine Speichereinrichtung an der Patientenwaage und/oder der Bildinformationsaufnahmeeinrichtung, die dazu angeordnet ist, von der Bildinformationsaufnahmeeinrichtung aufgezeichnete Bildinformationen in Form von zumindest einem Standbild und/oder zumindest einer Bewegtbildsequenz für eine Betrachtung abrufbar zu speichern, und eine Anzeigeeinrichtung an der Patientenwaage und/oder der Bildinformationsaufnahmeeinrichtung, die dazu angeordnet ist, von der Bildinformationsaufnahmeeinrichtung aufgezeichnete und von der Speichereinrichtung gespeicherte Standbilder und/oder Bewegtbildsequenzen anzuzeigen.

Damit wird vorteilhaft das Anzeigen und Speichern der gesamten Vorgänge als Video und/oder in Bildern unterstützt. Wiegevorgänge werden mittels Bildern, oder als Sequenz von Bildern, protokolliert und abgespeichert. Es kann beispielsweise erkannt werden, welche Pflegeperson den Wiegevorgang durchgeführt hat, oder es können manuell dokumentierte Protokolle anhand gespeicherter und später angezeigter Aufzeichnungen nachverfolgt und/oder nachvollzogen werden.

Bevorzugt ist die Anzeigeeinrichtung dabei dazu angeordnet, zumindest einen aufgezeichneten Wiegevorgang vor einer Dialysebehandlung des Patienten und zumindest einen aufgezeichneten Wiegevorgang nach der Dialysebehandlung des Patienten gleichzeitig und miteinander vergleichbar anzuzeigen.

Die Anzeigeeinrichtung kann hierzu beispielsweise mehrere Fenster oder Bereiche bereitstellen, in welche Aufnahmen unterschiedlicher Wiegevorgänge ladbar sind. Mittels etwa einer Zeitlinie können dann gewünschte Teile korrespondierender Wiegevorgänge oder Abläufe nebeneinander zur Anzeige gebracht und auf relevante Unterschiede und Ursachen von beispielsweise unerklärlichen Gewichtsunterschieden zwischen einem zeitlich früheren und einem zeitlich späteren Wiegevorgang vergleichend untersucht werden. Eine solche Untersuchung kann auch teilautomatisiert oder vollautomatisiert erfolgen.

Ein Dialysetherapiesystem zur Durchführung einer Dialysebehandlung mit Ermittlung eines Patientengewichts zumindest vor der Dialysebehandlung und/oder nach der Dialysebehandlung beinhaltet eine Gewichtserfassungseinrichtung wie vorstehend kurzbeschrieben, zumindest eine Dialysemaschine, die mit dem ggf. internen Datennetzwerk verbunden ist und dazu angeordnet ist, die Dialysebehandlung des Patienten durchzuführen; zumindest eine Arbeitsstation, die mit dem Datennetzwerk verbunden ist und zumindest einen Bildschirm und eine Tastatur beinhaltet, und zumindest eine Servereinheit, die mit dem Datennetzwerk verbunden ist und zumindest eine Datenbank mit für die Dialysebehandlung relevanten Daten vorhält, wobei die zumindest eine Bildinformationsaufnahmeeinrichtung ein Sichtfeld auf die Dialysemaschine und/oder die Patientenwaage zur visuellen Erfassung von Behandlungs- und/oder Wiegevorgängen aufweist und dazu angeordnet ist, innerhalb des Sichtfelds einen Ablauf der Dialysebehandlung und/oder des Wiegevorgangs derart aufzuzeichnen, dass die Ursache einer im Verlauf der Dialysetherapie auftretenden Änderung des Ist-Gewichts aus zumindest einer entsprechenden Aufzeichnung zumindest eines Wiegevorgangs ermittelbar ist.

Ein solches Dialysetherapiesystem bindet zusätzlich zu der kameraüberwachten Patientenwaage eine Reihe weiterer vernetzter Komponenten ein, wobei insbesondere Patientenwaage und Kamera über das Datennetzwerk mit einem Überwachungssystem und/oder einem Datenübernahme- und Auswertungssystem verbunden sind, welches eine automatisierte Auswertung der Aufnahmen, gegebenenfalls unter Hinzunahme weiterer Daten aus einer vernetzten Datenbank und/oder weiterer Parameter, mit Erweiterungen, anpassbaren Komponenten, Speicherplatz und/oder Rechenleistung und dergleichen unterstützt. Beispielsweise können Rollstühle und dergleichen über einen Barcode oder eine Farbcodierung identifizierbar sein. Ist ein Gewicht solcher Rollstühle in einer Datenbank hinterlegt, kann eine Abweichung im Hinblick auf Gewichte bzw. ein Gewichtsversatz oder Offset automatisch herausgerechnet werden.

Bei einem solchen Dialysetherapiesystem kann vorteilhaft zumindest eine Bildinformationsaufnahmeeinrichtung in der Patientenwaage eingebaut oder in deren Umgebung angeordnet mit Sicht auf wiegerelevante und ausgaberelevante Abschnitte der Patientenwaage vorgesehen sein, und kann zumindest eine Bildinformationsaufnahmeeinrichtung in der Dialysemaschine eingebaut oder in deren Umgebung angeordnet mit Sicht auf dialyserelevante und/oder wiegerelevante Abschnitte der Dialysemaschine und/oder der Patientenwaage vorgesehen sein, wobei zumindest eine der Bildaufnahmeeinrichtungen für eine Beobachtung des Patienten und/oder eine Videotelefonie-Verbindung zu dem Patienten eingerichtet ist.

Hierdurch wird es zum Beispiel möglich, mittels einer Kamera einen Wiegevorgang aufzuzeichnen und mittels einer anderen Kamera ein Videotelefonat zwischen einem behandelnden Arzt und einem behandelten Patienten zu führen. Dies erweist sich als vorteilhaft, wenn Befragungen des Patienten während eines Wiegevorgangs und/oder einer Dialysebehandlung durchgeführt werden sollen, um etwa dem Patienten ärztliche Anweisungen geben zu können oder Gewichtsunterschiede an Ort und Stelle aufzuklären, ohne dass hierzu der Arzt persönlich anwesend zu sein braucht. Insoweit kann die Dialysestation von personell erforderlicher Anwesenheit entlastet werden, und kann dennoch eine kurzfristige Verfügbarkeit ärztlicher Beteiligung und Entscheidung bereitgestellt werden.

Bevorzugt beinhaltet das Dialysetherapiesystem eine Speichereinrichtung in dem Datennetzwerk, die dazu angeordnet ist, von der Bildinformationsaufnahmeeinrichtung aufgezeichnete Bildinformationen in Form von zumindest einem Standbild und/oder zumindest einer Bewegtbildsequenz für eine Betrachtung abrufbar zu speichern, eine Auswerteeinrichtung, die dazu angeordnet ist, eine automatische Auswertung der aufgezeichneten Bildinformationen entsprechend vorgebbarer Parameter durchzuführen, und eine Anzeigeeinrichtung, die dazu angeordnet ist, von der Bildinformationsaufnahmeeinrichtung aufgezeichnete und von der Speichereinrichtung gespeicherte Standbilder und/oder Bewegtbildsequenzen anzuzeigen.

Damit wird vorteilhaft das Anzeigen, Auswerten und/oder Speichern der gesamten Vorgänge als Video und/oder in Bildern unterstützt. Wiegevorgänge werden mittels Bildern, oder als Sequenz von Bildern, protokolliert und abgespeichert. Es kann beispielsweise erkannt werden, welche Pflegeperson den Wiegevorgang durchgeführt hat, oder es können manuell dokumentierte Protokolle anhand gespeicherter und später angezeigter Aufzeichnungen nachverfolgt und/oder nachvollzogen werden. Dadurch, dass auch die Dialysemaschine mit zumindest einer Kamera in das System eingebunden ist, können softwaregestützt Bewegungen analysiert und Nahrungsaufnahmen erkannt werden, und kann eine Qualitätssicherung von Maßnahmen während der Dialyse, beispielsweise ob Medikamente verabreicht und auch eingenommen wurden, bereitgestellt und ausgewertet werden, oder kann erfasst werden, ob zu relevanten Bewegungen des Patienten auch entsprechende Wiegevorgänge dazu durchgeführt wurden und gültig vorliegen.

Vorteilhaft ist außerdem, wenn die vorgebbaren Parameter ein Gewicht des Patienten, eine Erkennung des Gesichts des Patienten, auf einer Patientenkarte gespeicherte Daten des Patienten, eine Nummer und/oder ein Zeitpunkt eines Wiegevorgangs, einen Barcode, und/oder eine Farbcodierung beinhalten, wobei die Parameter zum Erkennen vorbestimmter Vorgänge während der Dialysebehandlung und/oder zur Identifikation von an der Dialysebehandlung beteiligten Personen wählbar und/oder kombinierbar sind.

Dies ist insbesondere bei der Identifikation eines Patienten anhand einer Kombination aus Gewicht und Gesichtserkennung, oder bei der Ermittlung, ob Patientenkarte und Patient zueinander passen, also nicht etwa ein Patient auf einer fehlerhaften Grundlage behandelt wird, hilfreich. Durch eine geeignete Auswahl und/oder Kombination der Parameter kann verbessert auf eine Optimierung der Gewichtserfassung und/oder der Dialysebehandlung hin eingestellt werden, beispielsweise in Fällen, in denen bekannte, sich aber möglicherweise von Wiegevorgang zu Wiegevorgang und/oder von Dialysebehandlung zu Dialysebehandlung ändernde Werte, wie etwa Gewichte von Mal zu Mal benutzter, jeweils aber verschiedener Rollstühle, aus Ergebnissen herausrechenbar sind.

### Kurzbeschreibung der Zeichnungen

Die Erfindung wird nachstehend anhand eines bevorzugten Ausführungsbeispiels unter Bezugnahme auf die beigefügte Zeichnung näher beschrieben. Es zeigen:
Fig. 1 eine vereinfachte Darstellung eines vernetzten Dialysetherapiesystems mit einer vernetzten Patientenwaage und vernetzten Aufzeichnungskameras gemäß einem Ausführungsbeispiel;
Fig. 2 eine vereinfachte Darstellung eines Ablaufs eines Wiegevorgangs eines Patienten mit automatischer Videoaufzeichnung gemäß einem Ausführungsbeispiel für eine Dialysebehandlung;
Fig. 3 eine vereinfachte Darstellung eines Ablaufs eines Wiegevorgangs mit Videoaufzeichnung gemäß einem Ausführungsbeispiel in Zusammenwirkung mit einem verbundenen Datenüberwachungs- und Auswertungssystem; und
Fig. 4 eine vereinfachte Darstellung eines bekannten Ablaufs eines Wiegevorgangs unter Verwendung einer Protokollierung erfasster Daten auf Papier.

### Detaillierte Beschreibung bevorzugter Ausführungsbeispiele

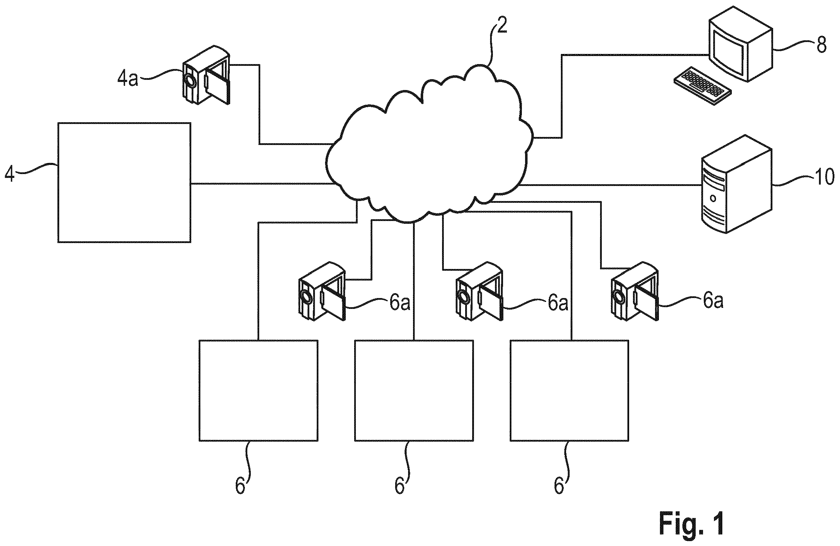

Fig. 1 zeigt eine vereinfachte Darstellung eines vorzugsweise vernetzten Gewichtsüberwachungssystems mit einer entsprechend vorzugsweise vernetzten Gewichtsüberwachungsvorrichtung gemäß einem Ausführungsbeispiel. Das Gewichtsüberwachungssystem und die Gewichtsüberwachungsvorrichtung werden bevorzugt auf einer Dialysestation zur Dialysebehandlung von Patienten mit Niereninsuffizienz eingesetzt. Die verwendete Gewichtsüberwachungsvorrichtung ist hierbei bevorzugt eine Patientenwaage an sich bekannter Art, mittels der das Gewicht eines Dialysepatienten vor, während und nach der Dialysetherapie ermittelbar ist. Das Gewichtsüberwachungssystem bildet zumindest Teil eines bevorzugt vernetzten Gesamtsystems, in welches auch die Dialysemaschinen selbst, sowie verschiedenartige unterstützende Personal- bzw. Arztplätze mit Datenstationen und/oder angebundenen Servereinrichtungen bzw. Rechner oder Computer eingebunden sein können.

Im Einzelnen zeigt Fig. 1 beispielhaft aber nicht darauf beschränkend ein Netzwerk oder Datennetzwerk 2, über welches die verschiedenen in das Gesamtsystem eingebundenen Geräte und Einrichtungen miteinander verbunden sind. Das Netzwerk 2 ist nicht auf eine bestimmte Netzwerkart oder darüber vernetzte Geräteart beschränkt und kann beispielsweise ein an sich bekanntes lokales Netzwerk (LAN), ein drahtloses Netzwerk (WLAN), ein Mobilfunk-Netzwerk, eine Kombination aus diesen oder eine einfache Verkabelung sein. Ebenfalls ist die Art der über das Netzwerk 2 übertragbaren Daten nicht auf eine bestimmte Datenform beschränkt. Insbesondere sind aber Bild- und Videodaten sowie für die Dialysebehandlung benötigte Daten in wahlfreier Richtung über das Netzwerk 2 sende- und empfangbar. Zusätzlich kann das Netzwerk 2 verschiedene Schnittstellen und Übergänge bereitstellen, beispielsweise einen Zugang zum Internet, zu einem Intranet einer Einrichtung, in deren Zuordnungsbereich sich die Dialysestation befindet, oder zu einem Fernbereichsnetzwerk (WAN) anderer Art, etwa einer verschiedene Kliniken, Forschungseinrichtungen, Universitäten, Labore und dergleichen vernetzenden Struktur.

Zumindest eine Patientenwaage 4 bildet eine Gewichtsermittlungsvorrichtung oder eine Gewichtserfassungsvorrichtung in dem Netzwerk 2. Die grundlegende Ausführungsform und Wiegefunktionen der Patientenwaage 4 sind an sich bekannt und ebenfalls nicht auf eine bestimmte Form und/oder Funktion beschränkt.

Fig. 1 zeigt ferner zumindest eine Dialysemaschine 6, mit welcher die Dialysebehandlung eines Patienten durchführbar ist. Beispielhaft sind drei in dem Netzwerk 2 vernetzte Dialysemaschinen 6 dargestellt.

Außerdem können zumindest eine Arbeits- oder Datenstation 8 mit zumindest einer Tastatur und einem Bildschirm, wie beispielsweise ein Client-Computer oder ein Terminal, der oder das sich an einem Personal- oder Arztplatz befinden kann, und zumindest ein Server-Computer 10, auf dem eine Datenbank und/oder Software und Rechenleistung für die Arbeitsstation 8 bereitgestellt sein kann, in dem Netzwerk 2 vorgesehen und vernetzt sein.

Mit dem Bezugszeichen 4a ist zumindest eine erste Bilderfassungseinrichtung oder Bildinformationsaufnahmeeinrichtung, mithin zumindest eine erste Kamera, bezeichnet. Die Kamera 4a ist in diesem Ausführungsbeispiel eine vernetzbare Netzwerk-Videokamera, d. h. eine Kamera, die mit dem Netzwerk 2 verbunden ist und zur Aufnahme von Still- und/oder Bewegtbildern innerhalb eines Aufnahme- oder Erfassungsbereichs beispielsweise eines Objektivs oder Bildsensors der Kamera sowie zu deren Weiterleitung an andere Empfangs- und/oder Weiterverarbeitungseinrichtungen in dem Netzwerk 2 ausgelegt ist. Die Kamera 4a kann dabei zur Erzeugung und/oder Verarbeitung von analogen und/oder digitalen Bilddaten eingerichtet sein, bevorzugt werden jedoch digitale Bilddaten erzeugt und genutzt.

Die erste Kamera 4a kann entweder direkt an der Patientenwaage 4 oder getrennt von dieser in einem Teil des Raumes an einer vorbestimmten Position angeordnet sein. Die Kamera 4a ist dabei insbesondere so angeordnet, dass ihr Aufnahmebereich für die Dialysetherapie relevante Vorgänge an der Patientenwaage 4, wie beispielsweise einen Stand des Patienten auf der Waage, äußerlich erkennbare Merkmale des Patienten, wie beispielsweise dessen Kleidung und Schuhe, einen Umgebungsbereich der Patientenwaage 4, in welchem sich der Patient möglicherweise abstützen kann, von dieser angezeigte Daten wie beispielsweise das ermittelte Gewicht des Patienten, und/oder den Verlauf des Wiegevorgangs, beispielsweise ob und wie der Patient sich bewegt, und dergleichen umfasst und erfassen kann. In anderen Worten ist die zumindest eine Kamera 4a derart angeordnet und ausgerichtet, dass mit ihr die Patientenwaage 4 beobachtet werden kann.

Mit dem Bezugszeichen 6a ist zumindest eine zweite Bilderfassungseinrichtung oder Bildinformationsaufnahmeeinrichtung, mithin eine zweite Kamera, bezeichnet. Die zweite Kamera 6a ist in diesem Ausführungsbeispiel ebenfalls eine vernetzbare Netzwerk-Videokamera, d. h. eine Kamera, die mit dem Netzwerk 2 verbunden ist und zur Aufnahme von Still- und/oder Bewegtbildern innerhalb eines Aufnahme- oder Erfassungsbereichs beispielsweise eines Objektivs oder Bildsensors der Kamera sowie zu deren Weiterleitung an andere Empfangs- und/oder Weiterverarbeitungseinrichtungen in dem Netzwerk 2 ausgelegt ist. Die Kamera 6a kann dabei zur Verarbeitung von analogen und/oder digitalen Bilddaten eingerichtet sein, bevorzugt werden jedoch digitale Bilddaten genutzt.

Die Kamera 6a kann entweder direkt an der Dialysemaschine 6 oder getrennt von dieser in einem Teil des Raumes an einer vorbestimmten Position angeordnet sein. Es kann jeweils eine dedizierte Kamera 6a für jede von mehreren Dialysemaschinen 6 oder eine Kamera 6a für mehrere Dialysemaschinen 6 vorgesehen sein. Die Kamera 6a ist dabei insbesondere so angeordnet, dass ihr Aufnahmebereich für die Dialysetherapie relevante Vorgänge an der Dialysemaschine 6, wie beispielsweise einen Zustand oder ein Verhalten eines an die Maschine angeschlossenen Patienten, äußerlich erkennbare Merkmale des Patienten, wie beispielsweise dessen Kleidung und Schuhe, einen Umgebungsbereich der Dialysemaschine 6, von dieser angezeigte Daten, und/oder den Verlauf des Dialysevorgangs und dergleichen umfasst und erfassen kann. In anderen Worten ist die zumindest eine zweite Kamera 6a derart angeordnet und ausgerichtet, dass mit ihr eine oder mehrere der Dialysemaschinen 6 beobachtet werden können.

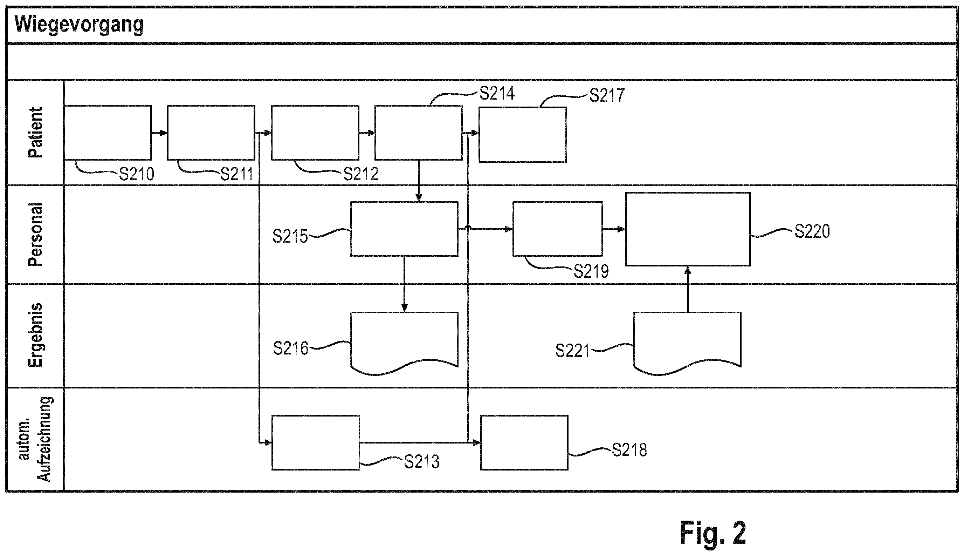

Nachstehend wird ein zeitlicher Ablauf eines Wiegevorgangs mit einer automatischen Bild- und/oder Videoaufzeichnung gemäß einem Ausführungsbeispiel für eine Dialysebehandlung näher beschrieben. Fig. 2 zeigt, auszugsweise anhand von vor der Dialysebehandlung durchzuführenden Schritten bzw. Aktivitäten, eine vereinfachte Darstellung eines solchen Ablaufs. Auszugsweise bedeutet, dass sich entsprechende Schritte und Aktivitäten auch während der auf den Vorbereitungsabschnitt folgenden Dialysebehandlung und auch nach deren Ende anschließen können.

Es wird angemerkt, dass der in Fig. 2 gezeigte Wiegevorgang vollständig manuell gesteuert werden kann, alternativ aber auch zumindest teilweise automatisiert ablaufen kann. Insbesondere kann hierbei die automatische Bild- und/oder Videoaufzeichnung manuell oder ereignisgesteuert, beispielsweise basierend auf Signalisierungen der Patientenwaage 4 oder auf Mechanismen einer Gestenerkennung, zum Beispiel Deut- und/oder Winkbewegungen vorbestimmter Art und/oder Richtung, in Gang gesetzt und/oder beendet werden, während etwa die Aufnahme sowie eine Übertragung und Weiterleitung erzeugter Bild- und/oder Videodaten über das Netzwerk 2 an eine nachgeordnete Weiterverarbeitungseinrichtung, z.B. den Server 10 oder die Arbeitsstation 8, und eine dortige Aufbereitung der Daten automatisiert erfolgen können.

In dem in Fig. 2 gezeigten Ablauf sind in chronologischer Abfolge und ausschnittsweise Schritte oder Aktivitäten angegeben, die während des Wiegevorgangs eines Patienten, beispielsweise eines Dialysepatienten mit Niereninsuffizienz, im Rahmen eines Therapieablaufs mit vorbereitendem Wiegen vor der Therapie von dem Patienten und Personal einer in diesem Beispielfall Dialysestation durchzuführen sind. Insoweit Aktivitäten und/oder Schritte parallel und/oder gleichzeitig durchzuführen sind, ist die Nummerierung der einzelnen Schritte nicht zwingend als Abfolge oder Aufeinanderfolge zu verstehen, sondern kann aus Zweckmäßigkeitsgründen auch nur eine Angabe von Bezugszeichen zur eindeutigen Zuordnung darstellen.

In einem Schritt S210 beginnt der Vorbereitungsablauf damit, dass beispielsweise der Patient in der Dialysestation eintrifft und für das Wiegen vorbereitende Maßnahmen wie etwa ein vorbestimmtes Ablegen von Kleidung, Schuhen, Gepäck und dergleichen durchführt. In einem Schritt S211 betritt der Patient sodann die Patientenwaage 4, oder nimmt auf ihr Platz. Gegebenenfalls kann dieser Vorgang von Personal der Dialysestation helfend unterstützt werden. Nachdem sich der Patient auf der Patientenwaage 4 befindet, wird der Wiegevorgang eingeleitet. Dies kann durch den Patienten selbst, durch das Personal, oder automatisch erfolgen. Automatisch beispielsweise dadurch, dass die für die Patientenwaage 4 zuständige erste Kamera 4a nach Art eines Direkt- oder Live-Bilds den Patienten auf der Patientenwaage 4 bereits erfasst, ohne bereits Bilddaten aufzuzeichnen, und auf die Erfassung beispielsweise einer als Start- oder Bereit-Signal interpretierbaren, vorbestimmten Geste des Patienten oder des Personals hin den Wiegevorgang und/oder dessen Aufzeichnung in Gang setzt. Möglichst zeitgleich mit dem in Gang setzen des Wiegevorgangs wird in einem Schritt S213 eine Aufzeichnung der von der Kamera 4a erzeugten und gelieferten Bildsignale und/oder Bilddaten begonnen. Eine solche Aufzeichnung dauert vorzugsweise solange ununterbrochen an, bis ein definiertes Ende des Wiegevorgangs signalisiert und/oder erfasst wird. Während die Aufzeichnung des Wiegevorgangs andauert, wird von dem Patienten in einem Schritt S212 erwartet, dass er während des Wiegens im Hinblick auf ein verlässliches und genaues Wiegeergebnis zumindest für eine vorbestimmte Zeitspanne stillsteht. Nach Ablauf des eigentlichen Wiegens des Patienten, welches beispielsweise durch die Patientenwaage 4 nach Verstreichen einer vorbestimmten, ausreichenden Zeitspanne signalisiert werden kann, wird in einem Schritt S214 das ermittelte oder festgestellte Gewicht an der Waage ausgelesen oder abgelesen und in einem Schritt S215 von dem Personal dokumentiert, beispielsweise in einem Schritt S216 in einem Protokoll festgehalten. In einem Schritt S218 wird sodann die Aufzeichnung durch die Kamera 4a angehalten oder beendet, und danach kann in einem Schritt S217 der Patient die Patientenwaage 4 wieder verlassen. Sodann ermittelt in einem Schritt S219 das Personal aus dem in Schritt S215 dokumentierten Gewicht das Ultrafiltrationsvolumen bzw. die Ultafiltrationsmenge und überträgt diese(s) in einem Schritt S220 als einen von Behandlungsparametern für die Dialysebehandlung in das Dialysegerät 6. Erforderliche Behandlungsparameter können von dem Personal in einem Schritt S221 auch aus dem Protokoll entnommen oder aus diesem abgelesen werden.

Dadurch, dass der Wiegevorgang an der Patientenwaage 4 durch den Aufzeichnungsvorgang mittels der Kamera4a überwacht, aufgezeichnet und für eine späteren Wiedergabe und/oder Weiterverarbeitung abgespeichert wird, wird der gesamte relevante Wiegevorgang über einen zweiten Protokollweg unabhängig von den Aktivitäten des Personals zusätzlich dokumentiert. Werden zu einem Zeitpunkt Abweichungen oder Unstimmigkeiten festgestellt, die einer Überprüfung bedürfen, kann auf die durch die Aufzeichnung bereitgestellte zusätzliche Dokumentation zugegriffen werden.

Beispielsweise kann die Kamera so ausgerichtet sein, dass ein Bild oder eine Aufzeichnung des Patienten zur Verfügung gestellt wird, während er die Waage betritt. Anhand der Aufzeichnung wird nachvollziehbar oder überprüfbar, ob der Patient vorschriftsmäßig Kleidung, Jacke, Schuhe und dergleichen abgelegt hatte, bevor der Wiegevorgang begonnen hat. Mehrere zeitlich beabstandete Aufzeichnungen können beispielsweise Aufschluss darüber liefern, ob zwei in Zusammenhang stehende oder in Zusammenhang zu bringende Wiegevorgänge unter unterschiedlichen Voraussetzungen stattgefunden haben. Falls beispielsweise das Tragen von Schuhen während des Wiegevorgangs zulässig ist, kann festgestellt werden, ob ein Wiegevorgang mit schweren Schuhen und ein anderer Wiegevorgang mit leichten Schuhen durchgeführt wurde.

Ist die Aufzeichnung der Kamera mit einem Datums- und/oder Zeitstempel versehen, können Abweichungen, die auf die Tageszeit zurückzuführen sind, wie beispielsweise ein Wiegevorgang am Morgen oder ein Wiegevorgang am Abend, ermittelt werden.

Ferner können anhand der Aufzeichnung mittels der Kamera 4a Ablesefehler durch das Personal ermittelt werden. Ist die Kamera so ausgerichtet, dass eine Anzeige, beispielsweise eine Gewichtsanzeige in Kilogramm auf einer Anzeigevorrichtung der Patientenwaage 4 oder ein Ausschlag oder ein Gleichgewichtszustand an einer Skala der Patientenwaage aufgezeichnet wird, kann im Nachhinein festgestellt werden, ob durch das Personal bei der Dokumentation des Patientengewichts in Schritt S215 korrekt abgelesen und/oder eine Ablesung in Schritt S216 korrekt in das Protokoll übertragen wurde. Wird zusätzlich eine Aufzeichnung des Protokolls, etwa als Videosequenz oder Standbild, angefertigt, kann ferner in Schritt S220 geprüft werden, ob ein aus dem Protokoll entnommener und in die Dialysemaschine 6 übertragener Behandlungsparameter korrekt abgelesen wurde.

Um derartige unterschiedliche Aspekte des Wiegevorgangs in eine Aufzeichnung einzubinden, kann insbesondere vorgesehen sein, dass die Kamera 4a bzw. ein Aufnahmeabschnitt derselben derart manuell und/oder motorisch schwenkbar und/oder zwischen einer Naheinstellung und einer Ferneinstellung oder Teleeinstellung verfahrbar ist. Dazu kann vorgesehen sein, dass die Kamera 4a entsprechend einem Zeitverlauf des Wiegevorgangs zu diesem korrespondierend nachgeführt wird, etwa durch Programmroutinen einer Kamerasteuerung, die auf eine Gestensteuerung ansprechen, nach Art eines Schaltwerks automatisch fortschreitend und/oder wechselnd, oder auf der Grundlage von Befehlen, die der Kamera über das Netzwerk 2 zugeführt werden.

Bei einer manuellen Zustandsänderung der Kamera 4a ist ferner denkbar, den Wiegevorgang in vorbestimmte Abschnitte einzuteilen, wie beispielsweise "Wiegebeginn", "Ablesung" und dergleichen, und die Patientenwaage 4 so mit der Kamera 4a zu koppeln, dass der Wiegevorgang nach Erreichen des Endes eines Abschnittes erst dann fortgeführt werden kann, nachdem die Kamera 4a auf eine neue Ausrichtung oder Einstellung geändert wurde. Beispielsweise kann dabei vorgesehen sein, dass die Patientenwaage 4 einen ermittelten und auszugebenden Wert erst dann zur Anzeige bringt, wenn sie ein Bestätigungssignal oder eine bestätigende Aktivität des Personals dahingehend erfasst hat, dass die Kamera 4a zur Aufzeichnung der Anzeige bzw. des angezeigten Werts ausgerichtet wurde und bereit ist.

Insoweit erfolgt gemäß Fig. 2 grundlegend eine Aufzeichnung von zumindest für eine Dokumentation, Nachprüfung, Nachvollziehbarkeit und, da die Aufzeichnung des Wiegevorgangs vorzugsweise vor der Übertragung von Behandlungsparametern in die Dialysemaschine 6 angehalten wird, gegebenenfalls Korrektur solcher Behandlungsparameter relevanter Abschnitte des Wiegevorgangs eines Patienten auf der Patientenwaage 4. Gegebenenfalls kann aber die Übertragung der Behandlungsparameter ebenfalls mit aufgezeichnet werden. Hierzu kann beispielsweise die Aufzeichnung nach Schritt S218 erneut gestartet und nach Schritt S220 erneut beendet werden, falls der Aufnahmebereich der Kamera 4a auch eine Behandlungsparameter-Eingabeumgebung umfasst oder die Kamera 4a zuvor entsprechend ausgerichtet wurde. Alternativ kann die Übertragung der Behandlungsparameter in die Dialysemaschine 6 auch von der dort vorgesehenen Kamera 6a aufgezeichnet werden.

Ein Speicherort der Aufzeichnung der Kamera 4a ist nicht auf einen bestimmten Speicherort beschränkt. Beispielsweise kann die Speicherung der Aufzeichnung lokal in der Kamera 4a selbst, etwa auf einem mit der Kamera 4a verbundenen Datenträger wie beispielsweise einer wechselbaren Speicherkarte, einem internen Datenträger oder einem von extern verbindbaren Datenträger erfolgen. Eine Kopie oder Sicherungskopie der Aufzeichnung kann jederzeit über das Netzwerk 2 an einem zusätzlichen Speicherort erstellt werden. Die Speicherung der Aufzeichnung kann alternativ auch unmittelbar durch Übertragung der erzeugten Bild- und/oder Videodaten über das Netzwerk 2 an eine vorbestimmte Speichereinrichtung erfolgen, etwa bei Verwendung einer speicherlosen Kamera 4a. Alternativ kann eine Speicherung der Aufzeichnung auch in einer entsprechend dazu ausgelegten und mit einer Speichereinrichtung und/oder einer zur Wiedergabe der Aufzeichnung eingerichteten Anzeigeeinrichtung versehenen Patientenwaage 4 erfolgen. Vorteilhaft ist hierbei, dass ein Wiegevorgang und dessen Dokumentation in Bild und Video an Ort und Stelle verfügbar sind, so dass bedarfsweise eine sehr schnelle Kontrolle erfolgen kann, während sich zum Beispiel der Patient noch in der Nähe der Waage befindet und sich Wiegebedingungen etwa durch eine zwischenzeitliche Nahrungsaufnahme und dergleichen nicht wesentlich geändert haben, und gegebenenfalls ein Wiegevorgang sehr schnell wiederholt werden kann.

Nachstehend wird ein zeitlicher Ablauf eines Wiegevorgangs mit einer automatischen Bild- und/oder Videoaufzeichnung gemäß dem Ausführungsbeispiel für eine Dialysebehandlung in Zusammenwirkung mit einem verbundenen Datenübernahme- und Auswertungssystem näher beschrieben.

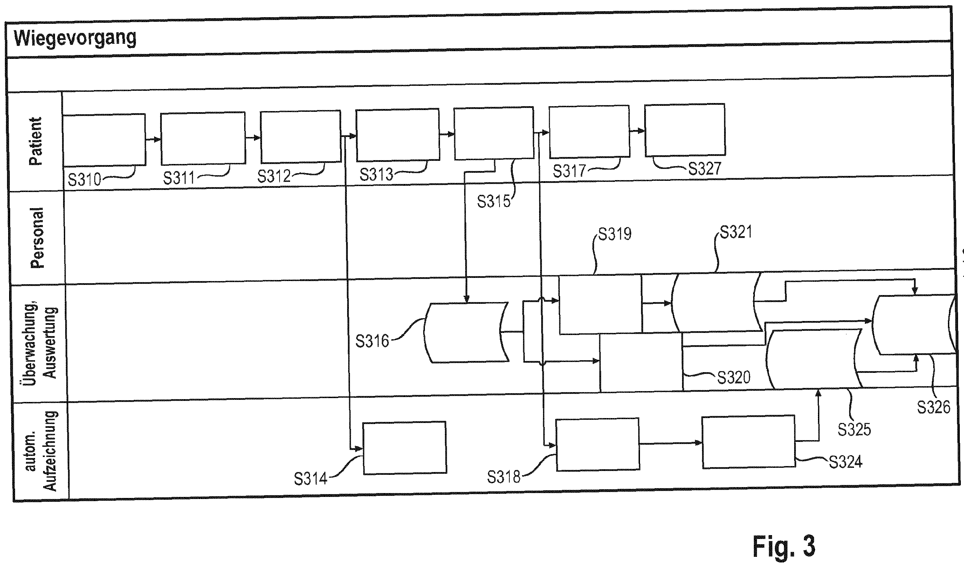

Fig. 3 zeigt, auszugsweise anhand von vor der Dialysebehandlung durchzuführenden Schritten bzw. Aktivitäten, eine vereinfachte Darstellung eines solchen Ablaufs. Auszugsweise bedeutet, dass sich entsprechende Schritte und Aktivitäten auch während der auf den Vorbereitungsabschnitt folgenden Dialysebehandlung und auch nach deren Ende anschließen können.

Es wird angemerkt, dass der in Fig. 3 gezeigte Ablauf und Wiegevorgang vollständig manuell gesteuert werden kann, alternativ aber auch teilautomatisiert bis hin zu vollautomatisiert ablaufen kann. Insbesondere kann die automatische Bild- und/oder Videoaufzeichnung manuell oder ereignisgesteuert, beispielsweise basierend auf Signalisierungen der Patientenwaage 4 oder auf Mechanismen einer Gestenerkennung, in Gang gesetzt und/oder beendet werden, während etwa die Aufnahme sowie eine Übertragung und Weiterleitung erzeugter Bild- und/oder Videodaten über das Netzwerk 2 an eine nachgeordnete Weiterverarbeitungseinrichtung, z.B. den Server 10 oder die Arbeitsstation 8, und eine dortige Aufbereitung der Daten automatisiert erfolgen können.

In dem in Fig. 3 gezeigten Ablauf sind in chronologischer Abfolge und ausschnittsweise Schritte oder Aktivitäten angegeben, die während des Wiegevorgangs eines Patienten, beispielsweise eines Dialysepatienten mit Niereninsuffizienz, im Rahmen eines Therapieablaufs mit vorbereitendem Wiegen vor der Therapie von dem Patienten und Personal einer in diesem Beispielfall Dialysestation durchzuführen sind. Insoweit Aktivitäten und/oder Schritte parallel und/oder gleichzeitig durchzuführen sind, ist die Nummerierung der einzelnen Schritte nicht zwingend als Abfolge oder Aufeinanderfolge zu verstehen, sondern kann aus Zweckmäßigkeitsgründen auch nur eine Angabe von Bezugszeichen zur eindeutigen Zuordnung darstellen.

Das in Fig. 3 insbesondere mitwirkende Datenübernahme- und Auswertungssystem kann hierbei eine über das Netzwerk 2 verbundene Software und Datenbank zur transparenten Abbildung und Steuerung von Vorgängen einer Dialysebehandlung sein. Ein grundlegende Ansätze und Funktionen bietendes System der Anmelderin ist bereits aus dem Stand der Technik hinlänglich bekannt und kann beispielsweise an der Arbeitsstation 8 und/oder dem Server 10 installiert oder über diese anderweitig zugänglich sein.

Im Wesentlichen übernimmt ein solches Datenübernahme- und Auswertungssystem Daten von mit ihm verbundenen Geräten, wie beispielsweise von der Patientenwaage 4 ermittelte Gewichte, von (nicht gezeigten) Analysegeräten während eines Überwachungs- bzw. Monitoring-Betriebsablaufs, und betreibt darüber hinaus eine Datenbank, über welche Zugriff auf Daten und Einträge von beispielsweise Krankenhausinformationssystemen, Laboren, Verwaltungen, externen Kliniken und Ärzte und dergleichen besteht. Ein in Abhängigkeit einer jeweiligen Funktionalität unidirektionaler oder bidirektionaler Datenaustausch zwischen angeschlossenen Geräten, der Datenübernahme/Datenerfassung/Datenüberwachung, der Datenbank, Arbeitsstationen oder Eingabeeinrichtungen und Lieferanten und/oder Benutzern der Daten der Datenbank ist möglich.

Fig. 3 stellt in einer Zeile "Überwachung, Auswertung" grundlegende Funktionen und Aufgaben dar, die gemäß dem Ausführungsbeispiel von dem Datenübernahme- und Auswertungssystem nach der Übernahme von Daten aus der Patientenwaage 4 in einem Schritt S316 und der Aufzeichnung und Aufbereitung des Wiegevorgangs in Schritten S314, S318 und S324 übernommen werden. Der Wiegevorgang und die Aufzeichnung werden dabei wie vorstehend unter Bezugnahme auf Fig. 2 beschrieben durchgeführt. Auch die Funktionen der Kamera 4a sind bis auf nachstehend dargestellte Unterschiede wie unter Bezugnahme auf Fig. 2 beschrieben.

Im Einzelnen beginnt der Ablauf nach Fig. 3 in einem Schritt S310. In einem Schritt S311 identifiziert sich der Patient an der Patientenwaage 4, beispielsweise mittels einer Chipkarte, einer Patientenkarte oder dergleichen, die von ihm oder von Personal in eine Kartenlesevorrichtung eingeführt wird. Das Datenübernahme- und Auswertungssystem erkennt den Patienten anhand dessen Identifikation und stellt gegebenenfalls bereits vorhandene Patientendaten aus der Datenbank bereit, oder kann einen neuen Patienteneintrag unter Verwendung der auf der Karte gespeicherten Daten anlegen. In einem Schritt S312 betritt der Patient die Patientenwaage 4 bzw. nimmt auf ihr Platz. Der Schritt S312 entspricht hierbei dem Schritt S211 in Fig. 2. In einem Schritt S313, der dem Schritt S212 in Fig. 2 entspricht, und einem Schritt S314, der dem Schritt S213 in Fig. 2 entspricht, wird der Wiegevorgang durchgeführt und dessen Aufzeichnung begonnen. In einem Schritt S315 wird anders als in Fig. 2 das von der Patientenwaage 4 gemessene oder ermittelte Gewicht, in diesem Ausführungsbeispiel das Ist-Gewicht, des Patienten nicht an der Patientenwaage 4 abgelesen, sondern an das Datenübernahme- und Auswertungssystem übertragen. Natürlich kann nach wie vor eine zusätzliche und ablesbare Anzeige des Gewichts an der Patientenwaage 4 vorgesehen sein. Nach Fig. 3 wird das Gewicht aber unmittelbar in das System übernommen und kann über dieses, vorzugsweise in zumindest nahezu Echtzeit, an einem Bildschirm dargestellt und abgelesen werden. Durch die direkte Übernahme in das System erfolgen auch unmittelbar eine sofortige Dokumentation des Gewichts und eine entsprechende Protokollierung, so dass in Fig. 3 die entsprechenden Schritte in Fig. 3 entfallen können. In einem Schritt S317, der dem Schritt S217 in Fig. 2 entspricht, verlässt der Patient die Patientenwaage 4 und entnimmt in einem Schritt S327 seine ihn identifizierende Karte bzw. lässt diese durch Personal entnehmen, und in einem Schritt S318, der dem Schritt S218 in Fig. 2 entspricht, wird die Aufzeichnung des Wiegevorgangs beendet.

Nach dem Schritt S316, in dem das durch den Wiegevorgang ermittelte Ist-Gewicht des Patienten vorliegt, verzweigt das Datenübernahme- und Auswertungssystem dessen Weiterverarbeitung in einen aus Schritten S319 und S321 bestehenden ersten Verarbeitungszweig und in einen aus einem Schritt S320 bestehenden zweiten Verarbeitungszweig.

In Schritt S319 wird ausgehend von dem in Schritt S316 erhaltenen Ist-Gewicht des Patienten ein Ultrafiltrationsvolumen bzw. eine Ultrafiltrationsmenge vor der Dialyse kalkuliert oder berechnet. In Schritt S321 wird diese berechnete Ultrafiltrationsmenge als Datensatz für die Dialysebehandlung, die hier nicht weiter dargestellt ist, zur Verfügung gestellt.

In Schritt S320 führt das Datenübernahme- und Auswertungssystem nach dem Ende der Dialysebehandlung eine Überprüfung der Ultrafiltrationsmenge durch. Die tatsächlich benötigte Ultrafiltrationsmenge ist nach der Dialysebehandlung bekannt und kann mit der vor der Dialysebehandlung anhand des Ist-Gewichts des Patienten errechneten Ultrafiltrationsmenge verglichen werden. Nicht vernachlässigbare Abweichungen können so unmittelbar festgestellt werden.

Möglichst zeitgleich oder parallel zu den Schritten S319 bis S321 wird in einem Schritt S324 nach dem Anhalten der Aufzeichnung in Schritt S318 die aufgezeichnete Bild- und/oder Videosequenz geeignet aufbereitet, beispielsweise zeitgestempelt, formatgewandelt, komprimiert, und oder in von dem Datenübernahme- und Auswertungssystem verwendbare Videodatensequenzen und Standbild- bzw. Fotosequenzen des Wiegevorgangs oder vorbestimmter Abschnitte desselben unterteilt. In einem Schritt S325 übernimmt sodann das Datenübernahme- und Auswertungssystem die in Schritt S324 aufbereitete Bild- und/oder Videosequenz.

Sodann erzeugt das Datenübernahme- und Auswertungssystem in einem Schritt S326 ein Behandlungsprotokoll der vorangehenden Dialysebehandlung. In das Behandlungsprotokoll in Schritt S326 gehen zumindest der die vorausberechnete Ultrafiltrationsmenge enthaltende Datensatz aus Schritt S321, die Überprüfung der Ultrafiltrationsmenge nach der Dialysebehandlung gemäß Schritt S320, beispielsweise als Ergebnis eines Vergleichs oder als ein absoluter Wert, und die Videodaten und/oder Bild- bzw. Fotosequenz des Wiegevorgangs ein. Dabei kann beispielsweise in das Behandlungsprotokoll eine Verknüpfung zu den andernorts gespeicherten Bild- und Videosequenzen eingetragen werden, über welche die Aufzeichnung des Wiegevorgangs erforderlichenfalls aufrufbar ist.

In Zuordnung zu dem eindeutig identifizierten Patienten sind insbesondere über mehrere Behandlungen, d. h. Tage oder Wochen, hinweg erstellte Aufzeichnungen entsprechender Wiegevorgänge bereitstellbar. Durch das Aufzeichnen der Wiegevorgänge mittels der Kamera 4a werden somit die einzelnen Abläufe auch längerfristig nachvollziehbar.

Hierzu kann vorgesehen sein, dass das Datenübernahme- und Überwachungssystem Bild- und Videodaten in Form wenigstens zweier nebeneinander angeordneter Fenster, die beispielsweise jeweils Standbilder oder Videosequenzen anzeigen, darstellt, wobei in jedem Fenster unabhängig voneinander Daten ein und desselben Wiegevorgangs oder zweier zeitlich auseinander liegender Wiegevorgänge anzeigbar sind. Somit kann auch dann, wenn das System bereits bei einem ersten Wiegevorgang eine Abweichung des Ist-Gewichts von einem Ist-Gewicht vergangener Zeiträume erfasst, der Wiegevorgang kontrolliert werden. Auf diese Weise können Ursachen wie beispielsweise das Tragen schwerer Schuhe anstelle von leichten Schuhen, eine nicht abgelegte Jacke, eine mitgewogene Tasche, ein Abstützen während des Wiegens und dergleichen erkannt werden. Treten ferner Abweichungen beispielsweise erst nach einem zweiten Wiegevorgang auf, kann gegebenenfalls eine Befragung des Patienten entfallen, da die Wiegevorgänge vermittels des Systems auch ohne eine solche Befragung nachvollziehbar sind. Als Ursachen für Abweichungen erst nach einem zweiten Wiegen können beispielsweise unterschiedliche Kleidung während beider Wiegevorgänge, unterschiedliche Schuhe während beider Wiegevorgänge, oder Gepäck bei einem oder beiden Wiegevorgängen erkannt werden.

Somit wurde eine Gewichtserfassungseinrichtung zur Ermittlung eines Patientengewichts für eine Dialysebehandlung beschrieben, die beinhaltet: eine Patientenwaage 4, die dazu angeordnet ist, einen Wiegevorgang durchzuführen und dadurch ein Ist-Gewicht eines sich auf der Waage befindenden Patienten zu erfassen, einen Netzwerkanschluss, der dazu angeordnet ist, zumindest eine Information und/oder Daten betreffend die Ermittlung des Patientengewichts und/oder den Wiegevorgang erzeugende Komponente der Gewichtserfassungseinrichtung mit einem Datennetzwerk 2 zu verbinden, eine Ausgabeeinrichtung, die dazu angeordnet ist, das erfasste Ist-Gewicht des Patienten an der Patientenwaage 4 ablesbar und/oder in einem vorbestimmten Datenformat in das Datennetzwerk 2 auszugeben, zumindest eine Bildinformationsaufnahmeeinrichtung 4a, 6a, die ein Sichtfeld auf die Patientenwaage 4 zur visuellen Erfassung von Wiegevorgängen aufweist und dazu angeordnet ist, innerhalb des Sichtfelds einen Ablauf des Wiegevorgangs derart aufzuzeichnen, dass eine Ursache einer im Verlauf der Dialysetherapie auftretenden Änderung des Ist-Gewichts aus zumindest einer entsprechenden Aufzeichnung zumindest eines Wiegevorgangs ermittelbar ist. Ein vernetztes Dialysetherapiesystem oder Dialysebehandlungssystem beinhaltet eine solche Gewichtserfassungseinrichtung, wobei therapeutische Aspekte, Verfahren, Maßnahmen und Anwendungen innerhalb des Therapiesystems vorhanden sein können, aber als außerhalb der vorstehend beschriebenen Einrichtung bzw. Vorrichtung zur Gewichtsbestimmung mit begleitender Bild- und Videoaufzeichnung des Wiegevorgangs zu werten sind.

### Bezugszeichenliste

- 2: Netzwerk oder Datennetzwerk
- 4: Patientenwaage
- 4a: erste Bilderfassungseinrichtung
- 6: Dialysemaschine
- 6a: zweite Bilderfassungseinrichtung
- 8: Arbeits- oder Datenstation
- 10: Server-Computer

- S210: Start
- S211: Waage betreten
- S212: Stillstehen während Wiegen
- S213: Aufzeichnung starten
- S214: Gewicht ablesen
- S215: Gewicht dokumentieren
- S216: Protokoll
- S217: Waage verlassen
- S218: Aufzeichnung stoppen
- S219: UF Volumen errechnen
- S220: Behandlungsparameter ins Gerät übertragen

- S310: Start
- S311: Patient identifiziert sich an Waage
- S312: Waage betreten
- S313: Stillstehen während Wiegen
- S314: Aufzeichnung starten
- S315: Gewicht übertragen
- S316: Ist-Gewicht
- S317: Waage verlassen
- S318: Aufzeichnung stoppen
- S319: Kalkulation UF-Volumen (vor Dialyse)
- S320: Überprüfung ist UF (nach Dialyse)
- S321: UF Volumen als Datensatz verfügbar
- S325: Videodaten und Fotosequenz des Wiegevorgangs
- S326: Behandlungsprotokoll

- S410: Ankunft im Zentrum
- S411: Kleidung ablegen
- S412: Wiegen
- S413: Ist-Gewicht
- S414: Protokoll
- S415: Ermittlung Soll-UF (Trockengew. IST)
- S416: Therapiedaten eingeben
- S417: Dialyse mit verschriebenen Therapiedaten durchführen
- S418: Protokoll
- S419: Wiegen
- S420: Ist-Gewicht
- S421: Protokoll
- S422: Ankleiden
- S423: Übertragung in Datenbank
- S424: Zentrum verlassen

## Patentansprüche

1. Gewichtserfassungseinrichtung eines Dialysesystems mit der ein Patientengewicht für eine Dialysebehandlung ermittelt wird, mit folgenden Merkmalen:
eine Patientenwaage (4), die dazu ausgelegt ist, einen Wiegevorgang durchzuführen und dadurch ein Ist-Gewicht eines sich auf der Waage befindenden Patienten zu erfassen und;
eine Ausgabeeinrichtung, die dazu angeordnet ist, das erfasste Ist-Gewicht des Patienten an der Patientenwaage (4) ablesbar und/oder in einem vorbestimmten Datenformat in ein Datennetzwerk (2) auszugeben;
**dadurch gekennzeichnet dass**
die Gewichtserfassungseinrichtung zumindest eine Bildinformationsaufnahmeeinrichtung (4a, 6a), die zumindest ein Sichtfeld auf die Patientenwaage (4) zur visuellen Erfassung von Wiegevorgängen aufweist, wobei
das Sichtfeld einen für einen Wiegevorgang des Dialysepatienten relevanten Raumbereich und/oder Geräteteile umfasst, und wobei
die Wiegevorgänge vor, während und nach der Dialysebehandlung erfasst und abgespeichert werden um ein Trockengewicht eines Patienten zur korrekten Bemessung einer Ultrafiltrationsmenge zu ermitteln und wobei
die Bildinformationsaufnahmeeinrichtung (4a, 6a), weiter dazu angepasst ist, den Patienten anhand einer Kombination aus Gewicht und Gesichtserkennung zu identifizieren und innerhalb ihres Sichtfelds einen Ablauf des Wiegevorgangs und der Dialysebehandlung derart lückenlos aufzuzeichnen und zu analysieren, dass eine Ursache einer im Verlauf einer den Wiegevorgang und die Dialysebehandlung umfassenden Dialysetherapie auftretenden Veränderung des Trockengewichts aus zumindest einer entsprechenden Aufzeichnung des Wiegevorgangs und der Dialysebehandlung ermittelt werden kann.

2. Gewichtserfassungseinrichtung nach Anspruch 1, **gekennzeichnet durch**
- einen Netzwerkanschluss, der dazu angeordnet ist, zumindest eine Information und/oder Daten betreffend die Ermittlung des Patientengewichts und/oder den Wiegevorgang erzeugende Komponente der Gewichtserfassungseinrichtung mit einem Datennetzwerk (2) zu verbinden;

3. Gewichtserfassungseinrichtung nach Anspruch 1 oder 2, **dadurch gekennzeichnet, dass** die Bildinformationsaufnahmeeinrichtung (4a) eine Kamera mit Anschluss an das Datennetzwerk (2) und in die Patientenwaage (4) eingebaut ist.

4. Gewichtserfassungseinrichtung nach Anspruch 1 oder 2, **dadurch gekennzeichnet, dass** die Bildinformationsaufnahmeeinrichtung (6a) eine Kamera mit Anschluss an ein Datennetzwerk (2) und/oder an die Patientenwaage hat, die vorzugsweise außerhalb der Patientenwaage (4) an einer Dialysemaschine (6) angeordnet ist.

5. Gewichtserfassungseinrichtung nach einem der vorangehenden Ansprüche, **dadurch gekennzeichnet, dass** die Bildinformationsaufnahmeeinrichtung (4a, 6a) in Bezug auf ihr aufzeichenbares Sichtfeld verschwenkbar ist.

6. Gewichtserfassungseinrichtung nach einem der vorangehenden Ansprüche, **dadurch gekennzeichnet, dass** die Bildinformationsaufnahmeeinrichtung (4a, 6a) durch Gesten einer sich im Sichtfeld befindenden Person aktivierbar, verschwenkbar und/oder deaktivierbar ist, und/oder ein Beginn, eine Unterbrechung und/oder ein Ende einer Aufzeichnung durch Gesten einer sich im Sichtfeld befindenden Person steuerbar sind.

7. Gewichtserfassungseinrichtung nach einem der vorangehenden Ansprüche, **dadurch gekennzeichnet, dass** die Bildinformationsaufnahmeeinrichtung (4a, 6a) dazu angeordnet ist, zumindest außerhalb eines aufzuzeichnenden Wiegevorgangs ein Direktbild ihres Sichtbereichs zu erfassen und bereitzustellen.

8. Gewichtserfassungseinrichtung nach einem der vorangehenden Ansprüche, **gekennzeichnet durch**
eine Speichereinrichtung an der Patientenwaage (4) und/oder der Bildinformationsaufnahmeeinrichtung (4a, 6a), die dazu angeordnet ist, von der Bildinformationsaufnahmeeinrichtung (4a, 6a) aufgezeichnete Bildinformationen in Form von zumindest einem Standbild und/oder zumindest einer Bewegtbildsequenz für eine Betrachtung abrufbar zu speichern; und
eine Anzeigeeinrichtung an der Patientenwaage (4) und/oder der Bildinformationsaufnahmeeinrichtung (4a, 6a), die dazu angeordnet ist, von der Bildinformationsaufnahmeeinrichtung aufgezeichnete und von der Speichereinrichtung gespeicherte Standbilder und/oder Bewegtbildsequenzen anzuzeigen.

9. Gewichtserfassungseinrichtung nach Anspruch 8, **dadurch gekennzeichnet, dass** die Anzeigeeinrichtung dazu angeordnet ist, zumindest einen aufgezeichneten Wiegevorgang vor einer Dialysebehandlung des Patienten und zumindest einen aufgezeichneten Wiegevorgang nach der Dialysebehandlung des Patienten gleichzeitig und miteinander vergleichbar anzuzeigen.

10. Dialysetherapiesystem zur Durchführung einer Dialysebehandlung mit Ermittlung eines Patientengewichts zumindest vor der Dialysebehandlung und/oder nach der Dialysebehandlung, **gekennzeichnet durch**
- eine Gewichtserfassungseinrichtung nach einem der vorangehenden Ansprüche 1 bis 9;
- zumindest eine Dialysemaschine (6), die mit dem Datennetzwerk (2) verbunden ist und dazu angeordnet ist, die Dialysebehandlung des Patienten durchzuführen;
- zumindest eine Arbeitsstation (8), die mit dem Datennetzwerk (2) verbunden ist und zumindest einen Bildschirm und eine Tastatur beinhaltet;
- zumindest eine Servereinheit (10), die mit dem Datennetzwerk (2) verbunden ist und zumindest eine Datenbank mit für die Dialysebehandlung relevanten Daten vorhält; wobei
- die zumindest eine Bildinformationsaufnahmeeinrichtung (6a) ein Sichtfeld auf die Dialysemaschine und/oder die Patientenwaage (4) zur visuellen Erfassung von Behandlungs- und/oder Wiegevorgängen aufweist und dazu angeordnet ist, innerhalb des Sichtfelds einen Ablauf der Dialysebehandlung und/oder des Wiegevorgangs derart aufzuzeichnen, dass die Ursache einer im Verlauf der Dialysetherapie auftretenden Änderung des Ist-Gewichts aus zumindest einer entsprechenden Aufzeichnung zumindest eines Wiegevorgangs ermittelbar ist.

11. Dialysetherapiesystem nach Anspruch 10, **dadurch gekennzeichnet, dass** zumindest eine Bildinformationsaufnahmeeinrichtung (4a) in der Patientenwaage (4) eingebaut oder in deren Umgebung angeordnet mit Sicht auf wiegerelevante und ausgaberelevante Abschnitte der Patientenwaage (4) vorgesehen ist, und zumindest eine Bildinformationsaufnahmeeinrichtung (6a) in der Dialysemaschine (6) eingebaut oder in deren Umgebung angeordnet mit Sicht auf dialyserelevante und/oder wiegerelevante Abschnitte der Dialysemaschine (6) und/oder der Patientenwaage (4) vorgesehen ist, wobei zumindest eine der Bildaufnahmeeinrichtungen (4a, 6a) für eine Beobachtung des Patienten und/oder eine Videotelefonie-Verbindung zu dem Patienten eingerichtet ist.

12. Dialysetherapiesystem nach Anspruch 10 oder 11, **gekennzeichnet durch**
eine Speichereinrichtung in dem Datennetzwerk (2), die dazu angeordnet ist, von der Bildinformationsaufnahmeeinrichtung (4a, 6a) aufgezeichnete Bildinformationen in Form von zumindest einem Standbild und/oder zumindest einer Bewegtbildsequenz für eine Betrachtung abrufbar zu speichern;
eine Auswerteeinrichtung, die dazu angeordnet ist, eine automatische Auswertung der aufgezeichneten Bildinformationen entsprechend vorgebbarer Parameter durchzuführen; und
eine Anzeigeeinrichtung, die dazu angeordnet ist, von der Bildinformationsaufnahmeeinrichtung (4a, 6a) aufgezeichnete und von der Speichereinrichtung gespeicherte Standbilder und/oder Bewegtbildsequenzen anzuzeigen.

13. Dialysetherapiesystem nach Anspruch 12, **dadurch gekennzeichnet, dass** die vorgebbaren Parameter ein Gewicht des Patienten, eine Erkennung des Gesichts des Patienten, auf einer Patientenkarte gespeicherte Daten des Patienten, eine Nummer und/oder ein Zeitpunkt eines Wiegevorgangs, ein Barcode, und/oder eine Farbcodierung beinhalten, wobei die Parameter zum Erkennen vorbestimmter Vorgänge während der Dialysebehandlung und/oder zur Identifikation von an der Dialysebehandlung beteiligten Personen wählbar und/oder kombinierbar sind.

## Claims

1. A weight detection device of a dialyis system with which a patient's weight is determined for dialysis treatment, comprising the following features:
patient scales (4) adapted to carry out a weighing procedure and thus detect an actual weight of a patient standing on the scales; and
an output device adapted to output the actual weight of the patient detected on the patient scales (4) in a readable manner and/or in a predetermined data format to a data network (2);
**characterized in that**
the weight detection device comprises at least one image information recording device (4a, 6a) which comprises at least a field of view directed to the patient scales (4) for the visual detection of weighing procedures, wherein
the field of view comprises a room area and/or apparatus components relevant to a weighing procedure of the dialysis patient, and wherein
the weighing procedures are detected and stored before, during, and after the dyalisis treatment in order to determine a dry weight of a patient for correctly measuing an ultrafiltration quantity, and wherein
the image information recording device (4a, 6a) is furthermore configured to identify the patient on the basis of a combination of weight and face recognition and to record and evaluate a course of the weighing procedure and the dialysis treatment within the field of view in full without gaps such that a cause of a change in the dry weight occurring in the course of the dialysis therapy comprising the weighing procedure and the dialysis treatment can be determined from at least one appropriate recording of the weighing procedure and the dialysis treatment.

2. The weight detection device according to claim 1, **characterized by**
a network connection arranged to connect at least one component of the weight detection device, generating information and/or data related to determining the patient's weight and/or to the weighing procedure, to a data network (2).

3. The weight detection device according to claim 1 or 2, **characterized in that** the image information recording device (4a) comprises a camera which includes a connection to the data network (2) and is installed in the patient scales (4).

4. The weight detection device according to claim 1 or 2, **characterized in that** the image information recording device (6a) comprises a camera including a connection to a data network (2) and/or to the patient scales, said camera being arranged on a dialysis apparatus (6) preferably outside the patient scales (4).

5. The weight detection device according to one of the preceding claims, **characterized in that** the image information recording device (4a, 6a) can be swiveled with respect to its recordable field of view.

6. The weight detection device according to one of the preceding claims, **characterized in that** the image information recording device (4a, 6a) can be activated, swiveled, and/or deactivated by gestures of a person situated in the field of view, and/or a beginning, interruption and/or end of a recording procedure can be controlled by gestures of a person situated in the field of view.

7. The weight detection device according to one of the preceding claims, **characterized in that** the image information recording device (4a, 6a) is arranged to detect and provide a direct image of its visual range at least outside the weighing procedure to be recorded.

8. The weight detection device according to one of the preceding claims, **characterized by**
a storage means which is provided on the patient scales (4) and/or on the image information recording device (4a, 6a) and adapted to retrievably save image information in the form of at least one video still and/or at least one moving picture sequence, which has/have been recorded by the image information recording device (4a, 6a), for the purpose of observation; and
a display device which is provided on the patient scales (4) and/or on the image information recording device (4a, 6a) and adapted to display video stills and/or moving picture sequences recorded by the image information recording device and saved by the storage means.

9. The weight detection device according to claim 8, **characterized in that** the display device is adapted to display at least one recorded weighing procedure before the dialysis treatment of the patient and at least one recorded weighing procedure after the dialysis treatment of the patient simultaneously and in a comparable fashion.

10. A dialysis therapy system for carrying out dialysis treatment by determining a patient's weight at least before the dialysis treatment and/or after the dialysis treatment, **characterized by**
- a weight detection device according to any one of the preceding claims 1 to 9;
- at least one dialysis apparatus (6) which is connected to the data network (2) and adapted to carry out the dialysis treatment of the patient;
- at least one work station (8) which is connected to the data network (2) and comprises at least a screen and a keyboard;
- at least one server unit (10) which is connected to the data network (2) and holds at least one database comprising data relevant to the dialysis treatment; wherein
- the at least one image information recording device (6a) comprises a field of view directed to the dialysis apparatus and/or the patient scales (4) for visually detecting treatment and/or weighing procedures and is adapted to record the course of the dialysis treatment and/or of the weighing procedure within the field of view in such a manner that the cause of a change in the actual weight occurring in the course of the dialysis therapy can be determined from at least one appropriate recording of at least one weighing procedure.

11. The dialysis therapy system according to claim 10, **characterized in that** at least one image information recording device (4a) is installed in the patient scales (4) or provided in its vicinity so as to have a view onto weighing-relevant and output-relevant portions of the patient scales (4), and at least one image information recording device (6a) is installed in the dialysis apparatus (6) or provided in its vicinity so as to have a view onto dialysis-relevant and/or weighing-relevant portions of the dialysis apparatus (6) and/or of the patient scales (4), at least one of the image information recording devices (4a, 6a) being adapted for observing the patient and/or for a video conferencing connection to the patient.

12. The dialysis therapy system according to claim 10 or 11, **characterized by**
a storage means provided in the data network (2) and adapted to retrievably save image information in the form of at least one video still and/or at least one moving picture sequence, which has/have been recorded by the image information recording device (4a, 6a), for the purpose of observation;
an evaluation device arranged to carry out automatic evaluation of the recorded image information according to predefinable parameters; and
a display device adapted to display video stills and/or moving picture sequences recorded by the image information recording device (4a, 6a) and saved by the storage means.

13. The dialysis therapy system according to claim 12, **characterized in that** the predefinable parameters comprise a weight of the patient, identification of the face of the patient, patient data saved on a patient card, number and/or point in time of a weighing procedure, a barcode, and/or a color coding, with the option of the parameters being capable of being selected and/or combined for identifying predetermined operations during the dialysis treatment and/or for identifying persons involved in the dialysis treatment.

## Revendications

1. Dispositif de capture pondérale d'un système de dialyse avec lequel un poids d'un patient est déterminé pour un traitement par dialyse, comportant les caractéristiques suivantes :
un pèse-personne (4) qui est conçu pour réaliser un processus de pesée et pour capturer ainsi un poids réel d'un patient se trouvant sur le pèse-personne ;
un dispositif d'édition qui est agencé pour éditer le poids réel capturé du patient sur le pèse-personne (4) de façon lisible et/ou dans un format de données prédéterminé dans un réseau de données (2) ;
**caractérisé en ce que**
le dispositif de capture pondérale a au moins un dispositif de réception d'informations d'image (4a, 6a) qui présente au moins un champ de vision sur le pèse-personne (4) pour la capture visuelle de processus de pesée,
dans lequel
le champ de vision comprend une zone d'espace et/ou des parties d'appareil pertinents pour un processus de pesée du patient sous dialyse, et dans lequel les processus de pesée avant, pendant et après le traitement par dialyser sont capturés et stockés pour déterminer un poids sec d'un patient pour la mesure correcte d'une quantité d'ultrafiltration, et dans lequel
le dispositif de réception d'informations d'image (4a, 6a) est en outre adapté pour identifier le patient sur la base d'une combinaison de poids et de reconnaissance de visage et enregistrer et analyser intégralement dans son champ de vision, un déroulement du processus de pesée et du traitement de dialyse de telle sorte qu'une cause d'une modification du poids sec survenant au cours d'une thérapie par dialyse comprenant le processus de pesée et le traitement par dialyse peut être déterminée à partir d'au moins un enregistrement correspondant du processus de pesée et du traitement par dialyse

2. Dispositif de capture pondérale selon la revendication 1, **caractérisé par**
- une connexion au réseau qui est adaptée pour relier au moins une information et/ou des données concernant la détermination du poids du patient et/ou des composants générant le processus de pesée du dispositif de capture pondérale avec un réseau de données (2).

3. Dispositif de capture pondérale selon la revendication 1 ou 2, **caractérisé en ce que** le dispositif de réception d'informations d'image (4a) présente une caméra dotée d'une connexion au réseau de données (2) et est intégré dans le pèse-personne (4).

4. Dispositif de capture pondérale selon la revendication 1 ou 2, **caractérisé en ce que** le dispositif de réception d'informations d'image (6a) a une caméra dotée d'une connexion à un réseau de données (2) et/ou au pèse-personne, qui est agencée de préférence hors du pèse-personne (4) sur une machine de dialyse (6).

5. Dispositif de capture pondérale selon l'une des revendications précédentes, **caractérisé en ce que** le dispositif de réception d'informations d'image (4a, 6a) peut pivoter par rapport à son champ de vision enregistrable.

6. Dispositif de capture pondérale selon l'une des revendications précédentes, **caractérisé en ce que** le dispositif de réception d'informations d'image (4a, 6a) peut être activé, peut pivoter et/ou peut être désactivé par des gestes d'une personne se trouvant dans le champ de vision et/ou un début, une interruption, et/ou une fin d'un enregistrement peut être commandé(e) par des gestes d'une personne se trouvant dans le champ de vision.

7. Dispositif de capture pondérale selon l'une des revendications précédentes, **caractérisé en ce que** le dispositif de réception d'informations d'image (4a, 6a) est agencé pour capturer et mettre à disposition au moins en dehors d'un processus de pesée à enregistrer, une image directe de sa zone de vision.

8. Dispositif de capture pondérale selon l'une des revendications précédentes, **caractérisé par**
un dispositif de stockage sur le pèse-personne (4) et/ou sur le dispositif de réception d'informations d'image (4a, 6a) qui est agencé pour stocker des informations d'image enregistrées par le dispositif d'enregistrement d'informations d'image (4a, 6a) sous la forme d'au moins une image statique et/ou d'au moins une séquence d'images mobiles pouvant être consultées pour un examen ; et
un dispositif d'affichage sur le pèse-personne (4) et/ou sur le dispositif de réception d'informations d'image (4a, 6a) qui est agencé pour afficher des images statiques et/ou des séquences d'images mobiles enregistrées par le dispositif de réception d'informations d'image et stockées par le dispositif de stockage.

9. Dispositif de capture pondérale selon la revendication 8, **caractérisé en ce que** le dispositif d'affichage est agencé pour afficher en même temps et de façon comparable conjointement au moins un processus de pesée enregistré avant un traitement par dialyse du patient et au moins un processus de pesée enregistré après le traitement du patient.

10. Système de traitement par dialyse pour la réalisation d'un traitement par dialyse comportant la détermination d'un poids d'un patient au moins avant le traitement par dialyse et/ou après le traitement par dialyse, **caractérisé par**
- un dispositif de capture pondérale selon l'une des revendications 1 à 9 précédentes ;
- au moins une machine de dialyse (6) qui est reliée au réseau de données (2) et est agencée en outre pour réaliser le traitement de dialyse du patient ;
- au moins un poste de travail (8) qui est relié au réseau de données (2) et comporte au moins un écran et un clavier ;
- au moins une unité de serveur (10) qui est reliée au réseau de données (2) et comporte au moins une banque de données contenant des données pertinentes pour le traitement par dialyse ; dans lequel
- l'au moins un dispositif de réception d'informations d'image (6a) présente un champ de vision sur la machine de dialyse et/ou sur le pèse-personne (4) pour la capture visuelle de processus de traitement et/ou de pesée et est agencé pour enregistrer dans le champ de vision, un déroulement du traitement par dialyse et/ou du processus de pesée de telle sorte que la cause d'une modification du poids réel survenant au cours de la thérapie par dialyse peut être déterminée à partir d'au moins un enregistrement correspondant d'au moins un processus de pesée.

11. Système de thérapie par dialyse selon la revendication 10, **caractérisé en ce qu'**au moins un dispositif de réception d'informations d'image (4a) intégré dans le pèse-personne (4) ou agencé dans son environnement avec vue sur des sections du pèse-personne (4), pertinentes pour la pesée et pertinente pour l'édition, est prévu, et au moins un dispositif de réception d'informations d'image (6a) est intégré dans la machine de dialyse (6) ou dans son environnement avec vue sur des sections de la machine de dialyse (6) et/ou la pesée du patient (4) pertinentes pour la dialyse et/ou pertinente pour la pesée, dans lequel au moins l'un des dispositifs de réception d'image (4a, 6a) est conçu pour une observation du patient et/ou une liaison visiophonique à un patient.

12. Système de thérapie par dialyse selon la revendication 10 ou 11, **caractérisé par** un dispositif de stockage dans le réseau de données (2) qui est agencé pour stocker par le dispositif de réception d'informations d'image (4a, 6a) des informations d'image enregistrées sous la forme d'au moins une image statique et/ou au moins une séquence d'images mobiles pouvant être consultées pour un examen ;
un dispositif d'analyse qui est agencé pour réaliser une analyse automatique des informations d'image enregistrées de façon correspondant à un paramètre prédéfinissable ; et
un dispositif d'affichage qui est agencé pour afficher des images statiques et/ou des séquences d'images mobiles enregistrées par le dispositif de réception d'informations d'image (4a, 6a) et par le dispositif de stockage.

13. Système de thérapie par dialyse selon la revendication 12, **caractérisé en ce que** les paramètres prédéfinis comportent un poids du patient, une reconnaissance du visage du patient, des données du patient stockées sur une carte de patient, un numéro et/ou un moment d'un processus de pesée, un code-barres et/ou un codage couleur, dans lequel les paramètres de reconnaissance de processus prédéterminés peuvent être choisis et/ou combinés pendant le traitement par dialyse et/ou l'identification de personnes participant au traitement par dialyse.
